# EUROPEAN PATENT SPECIFICATION

(11) **EP 1 778 837 B1**
(45) Date of publication and mention of the grant of the patent: **16.04.2008**
(21) Application number: 04822235.0
(22) Date of filing: 24.11.2004
(51) Int. Cl.: C12N 9/02

(54) **TREATMENT OF NEURODEGENERATIVE DISEASES BY THE USE OF SCD4 INHIBITORS**
BEHANDLUNG NEURODEGENERATIVER KRANKHEITEN DURCH VERWENDUNG VON SCD4-INHIBITOREN
UTILISATION D'INHIBITEURS DE SCD4 POUR LE TRAITEMENT DE MALADIES DÉGÉNÉRATIVES

(30) Priority: 09.08.2004 EP 04018874; 02.09.2004 WO PCT/EP2004/009771
(43) Date of publication of application: 02.05.2007
(73) Proprietor: CELLZOME AG, 69117 Heidelberg (DE)
(72) Inventor: HOPF, Carsten, 68165 Mannheim (DE); DREWES, Gerard, 69121 Heidelberg (DE); RUFFNER, Heinz, 69245 Bammental (DE)
(74) Representative: Huhn, Michael
(86) International application number: PCT/EP2004/013340
(87) International publication number: WO 2006/015621

(56) References cited:
- WO-A-01/53468
- WO-A-01/66758
- WO-A-02/32286
- WO-A-02/062999
- URYU SHIGEKO ET AL: "beta-Amyloid-specific upregulation of stearoyl coenzyme A desaturase-1 in macrophages." BIOCHEMICAL AND BIOPHYSICAL RESEARCH COMMUNICATIONS. 28 MAR 2003, vol. 303, no. 1, 28 March 2003 (2003-03-28), pages 302-305, XP002323253 ISSN: 0006-291X
- MORLEY JOHN E ET AL: "Alzheimer's disease through the eye of a mouse. Acceptance lecture for the 2001 Gayle A. Olson and Richard D. Olson prize." PEPTIDES. MAR 2002, vol. 23, no. 3, March 2002 (2002-03), pages 589-599, XP002323254 ISSN: 0196-9781
- SUN YU ET AL: "Expression of liver X receptor target genes decreases cellular amyloid beta peptide secretion." THE JOURNAL OF BIOLOGICAL CHEMISTRY. 25 JUL 2003, vol. 278, no. 30, 25 July 2003 (2003-07-25), pages 27688-27694, XP002323255 ISSN: 0021-9258
- LI YI-JU ET AL: "Glutathione S-transferase omega-1 modifies age-at-onset of Alzheimer disease and Parkinson disease." HUMAN MOLECULAR GENETICS. 15 DEC 2003, vol. 12, no. 24, 15 December 2003 (2003-12-15), pages 3259-3267, XP002323256 ISSN: 0964-6906

## Description

The present invention relates to the role of SCD4 in APP-processing and the use of inhibitors of SCD4 in the treatment of neurogenerative diseases.

Alzheimer's disease is a chronic condition that affects millions of individuals worldwide.

The brains of sufferers of Alzheimer's disease - show a characteristic pathology of prominent neuropathologic lesions, such as the initially intracellular neurofibrillary tangles (NFTs), and the extracellular amyloid-rich senile plaques. These lesions are associated with massive loss of populations of CNS neurons and their progression accompanies the clinical dementia associated with AD. The major component of amyloid plaques are the amyloid beta (A-beta, Abeta or Aβ) peptides of various lengths. A variant thereof, which is the Aβ1-42-peptide (Abeta-42) is the major causative agent for amyloid formation. Another variant is the Aβ1-40-peptide (Abeta-40). Amyloid beta is the proteolytic product of a precursor protein, beta amyloid precursor protein (beta-APP or APP). APP is a type-I trans-membrane protein which is sequentially cleaved by several different membrane-associated proteases. The first cleavage of APP occurs by one of two proteases, alpha-secretase or beta-secretase. Alpha secretase is a metalloprotease whose activity is most likely to be provided by one or a combination of the proteins ADAM10 and ADAM17. Cleavage by alpha-secretase precludes formation of amyloid peptides and is thus referred to as non-amyloidogenic. In contrast, cleavage of APP by beta-secretase is a prerequisite for subsequent formation of amyloid peptides. This secretase, also called BACE1 (beta-site APP-cleaving enzyme), is a type-1 transmembrane protein containing an aspartyl protease activity (described in detail below).

The beta-secretase (BACE) activity cleaves APP in the ectodomain, resulting in shedding of secreted, soluble APPb, and in a 99-residue C-terminal transmembrane fragment (APP-C99). Vassar et al. (Science 286, 735-741) cloned a transmembrane aspartic protease that had the characteristics of the postulated beta-secretase of APP, which they termed BACE1. Brain and primary cortical cultures from BACE1 knockout mice showed no detectable beta-secretase activity, and primary cortical cultures from BACE knockout mice produced much less amyloid-beta from APP. This suggests that BACE1, rather than its paralogue BACE2, is the main beta-secretase for APP. BACE1 is a protein of 501 amino acids containing a 21-aa signal peptide followed by a proprotein domain spanning aa 22 to 45. There are alternatively spliced forms, BACE-1-457 and BACE-1-476. The extracellular domain of the mature protein is followed by one predicted transmembrane domain and a short cytosolic C-terminal tail of 24 aa. BACE1 is predicted to be a type 1 transmembrane protein with the active site on the extracellular side of the membrane, where beta-secretase cleaves APP and possible other yet unidentified substrates. Although BACE1 is clearly a key enzyme required for the processing of APP into A-beta, recent evidence suggests additional potential substrates and functions of BACE1 (J. Biol. Chem. 279, 10542-10550). To date, no BACE1 interacting proteins with regulatory or modulatory functions have been described.

The APP fragment generated by BACE1 cleavage, APP-C99, is a substrate for the gamma-secretase activity, which cleaves APP-C99 within the plane of the membrane into an A-beta peptide (such as the amyloidogenic Aβ1-42 peptide), and into a C-terminal fragment termed APP intracellular domain (AICD) (Annu Rev Cell Dev. Biol 19, 25-51). The gamma-secretase activity resides within a multiprotein complex with at least four distinct subunits. The first subunit to be discovered was presenilin (Proc Natl Acad Sci USA 94, 8208-13). Other known protein components of the gamma-secretase complex are Pen-2, Nicastrin and Aph-1a.

Despite recent progress in delineating molecular events underlying the etiology of Alzheimer's disease, no disease-modifying therapies have been developed so far. To this end, the industry has struggled to identify suitable lead compounds for inhibition of BACE1. Moreover, it has been recognized that a growing number of alternative substrates of gamma-secretase exist, most notably the Notch protein. Consequently, inhibition of gamma-secretase is likely to cause mechanism-based side effects. Current top drugs (e.g. Aricept®/donepezil) attempt to achieve a temporary improvement of cognitive functions by inhibiting acetylcholinesterase, which results in increased levels of the neurotransmitter acetylcholine in the brain. These therapies are not suitable for later stages of the disease, they do not treat the underlying disease pathology, and they do not halt disease progression.

Thus, there is an unmet need for the identification of novel targets allowing novel molecular strategies for the treatment of Alzheimer's disease. In addition, there is a strong need for novel therapeutic compounds modifying the aformentioned molecular processes by targeting said novel targets:

In a first aspect, the invention provides the use of a SCD4 inhibitor for the preparation of a pharmaceutical composition for the treatment of Alzheimer's disease, wherein the inhibitor is selected from the group consisting of antisense oligonucleotides, siRNA and ribozymes, and wherein the inhibitor modulates the activity of gamma secretase and/or beta secretase.

In the context of the present invention, using functional assays, it has been surprisingly found that SCD4 is a novel target enabling novel therapies for the treatment of Alzheimer's disease.

The identification of SCD4 as a key target molecule enables the use of SCD4 interacting molecules for the treatment of neurodegenerative diseases. This is especially shown in the Example-section (infra) where it is demonstrated that siRNA directed against SCD4 results in a lowered or attenuated secretion/generation of Abeta-42.

In the context of the present invention, a "SCD4 interacting molecule" is a molecule which binds at least temporarily to SCD4 and which preferably modulates SCD4 activity.

Stearoyl-CoA desaturase (SCD) is the rate-limiting enzyme in the biosynthesis of monounsaturated fatty acids. At least four isozymes exist in mouse (SCD1 to SCD4). The enzyme converts stearate (and palmitate) into monounsaturated fatty acids (mostly -oleate; C18:1).

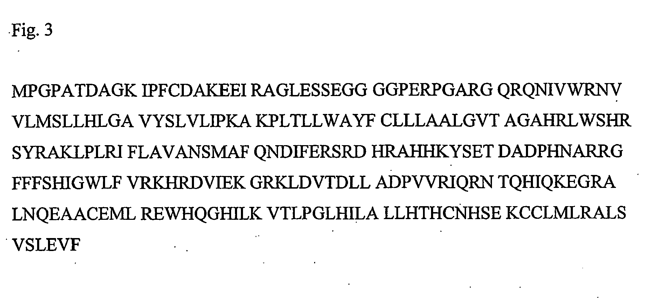

In humans, there seem to exist only two orthologous SCD genes (SCD1 and SCD4). Human SCD4 (see Fig. 3, also known as Hypothetical Protein FLJ21032) is similar to SCD1 in a sequence stretch containing the catalytic domain, but it is otherwise quite distinct. Human SCD4 is located on chromosome 4q21.3.

Recent evidence suggests that human SCD4 is a brain-specific enzyme in human fetuses and that the gene is disrupted in a family with cleft lip (Beiraghi S, Zhou M, Talmadge CB, Went-Sumegi N, Davis JR, Huang D, Saal H, Seemayer TA, Sumegi J (2003) Identification and characterization of a novel gene disrupted by a pericentric inversion inv(4)(p13.1q21.1) in a family with cleft lip. Gene. 309(1):11-21.). However, Beiraghi et al. found no expression of SCD4 in any adult human tissue.

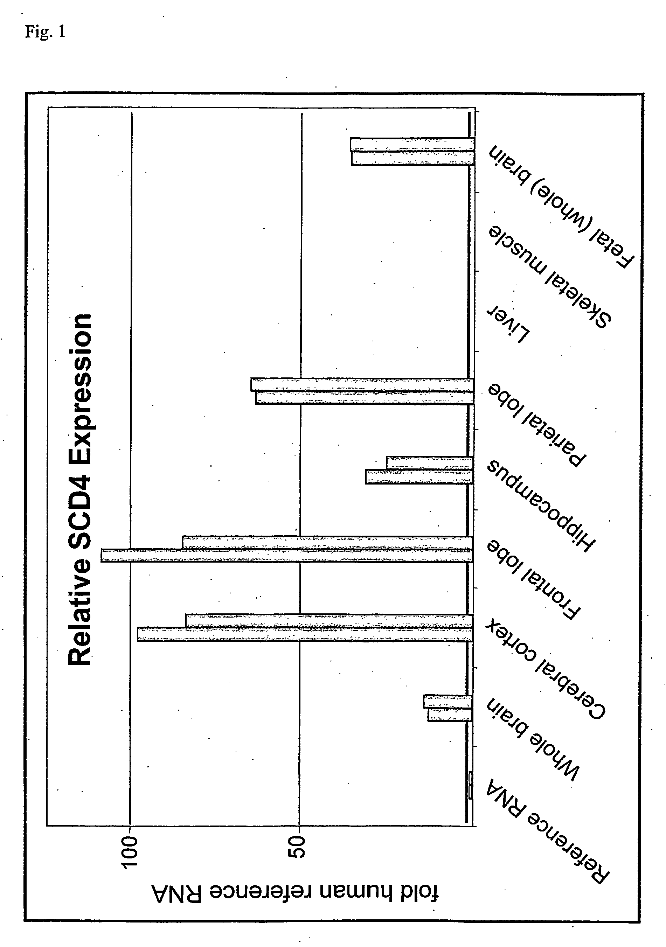

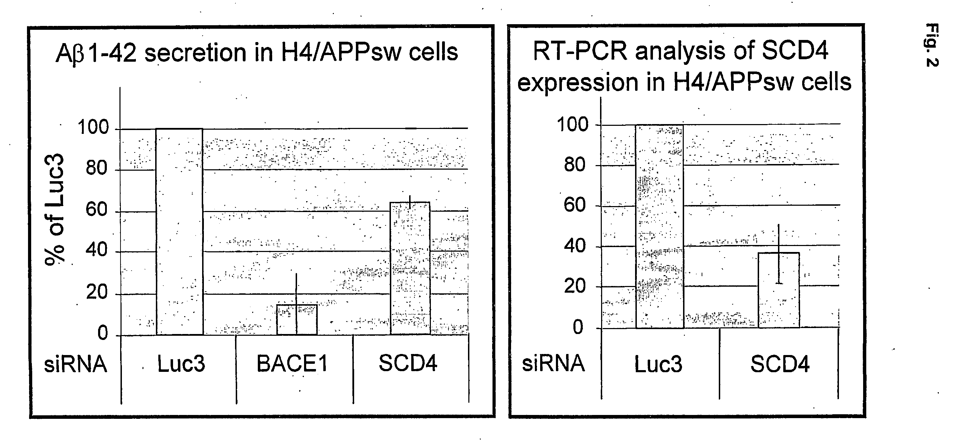

In contrast, in the context of the present invention, it was found that SCD4 is strongly expressed at much higher levels in adult human brain than in any other tissue analyzed (see Fig. 1).

According to the present invention, the expression "SCD4" does not only mean the protein as shown in Fig. 3, but also a functionally active derivative thereof, or a functionally active fragment thereof, or a homologue thereof, or a variant encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCI (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCI (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

Generally, the term "functionally active" as used herein refers to a polypeptide, namely a fragment or derivative, having structural, regulatory, or biochemical functions of the protein according to the embodiment of which this polypeptide, namely fragment or derivative is related to.

In the case of SCD4, a functionally active derivative preferably means a derivate which exerts essentially the same activity as SCD4, e.g. it converts stearate (and palmitate) into monounsaturated fatty acids (mostly oleate; C18:1) and/or it is capable of playing a similar role as SCD4 in Abeta-42 secretion.
The activity of SCD4 (as a Delta-9 desaturase activity) as well as of a functionally active derivative thereof can be measured as described in Obukowicz MG et at, The Journal of Pharmacology and Experimental Therapeutics (JPET) 287:157-166 (1998).

According to the present invention, the term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, stabilize, inhibit, suppress or destabilize the function of other molecules, stability, ability to localize to certain subcellular locations. Where applicable, said term also relates to lowering or attenuating the secretion of Abeta-42 if the molecule is inhibited.

According to the present invention, the terms "derivatives" or "analogs" of SCD4 or "variants" as used herein preferably include, but are not limited, to molecules comprising regions that are substantially homologous to the SCD4, in various embodiments, by at least 70%, 80%, 90%, 95% or 99% identity over an amino acid sequence of identical size, or when compared to an aligned sequence in which the alignment is done by a .computer homology program known in the art, or whose encoding nucleic acid is capable of hybridizing to a sequence encoding the protein under stringent, moderately stringent, or nonstringent conditions. The catalytic domain may be more highly conserved than a non-catalytic region of the protein. Therefore, the above percentages of identity may be higher if a region comprising, particularly consisting of, the entire or part of the catalytic domain is chosen (e.g. 85%, 90%, 95% or 99%), or it may be lower, if a region not comprising the catalytic domain is chosen (e.g. 30%, 40%, 50%, 60%, 70%). It means a protein which is the outcome of a modification of the naturally occurring protein, by amino acid substitutions, deletions and additions, respectively, which derivatives still exhibit the biological function of the naturally occurring protein although not necessarily to the same degree. The biological function of such proteins can e.g. be examined by suitable available in vitro assays as provided in the invention.

The term "fragment" as used herein refers to a polypeptide of at least 10, 20, 30, 40 or 50 amino acids of the protein, particularly SCD4, according to the embodiment. In specific embodiments, such fragments are not larger than 35, 100 or 200 amino acids.

The term "gene" as used herein refers to a nucleic acid comprising an open reading frame encoding a polypeptide of, if not stated otherwise, the present invention, including both exon and optionally intron sequences.

The terms "homologue" or "homologous gene products" as used herein mean a protein in another species, preferably mammals, which performs the same biological function as the protein described herein, in particular SCD4. Such homologues are also termed "orthologous gene products". The algorithm for the detection of orthologue gene pairs from humans and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g., in Nature, 2001, 409:860-921. The homologues of the proteins according to the invention can either be isolated based on the sequence homology of the genes encoding the proteins provided herein to the genes of other species by cloning the respective gene applying conventional technology and expressing the protein from such gene, or by other suitable methods commonly known in the art.

According to the present invention the term "SCD4-inhibitor" refers to a SCD4-interacting molecule being a biochemical or chemical compound which preferably inhibits or reduces the activity of SCD4. This can e.g. occur via suppression of the expression of the corresponding gene. The expression of the gene can be measured by RT-PCR or Western blot analysis. Furthermore, this can occur via inhibition of the activity, e.g. by binding to SCD4.

Examples of such SCD4 inhibitors are binding proteins or binding peptides directed against SCD4, in particular against the active site of SCD4, and nucleic acids directed against the SCD4 gene.

The term "nucleic acids against SCD4" refers to double-stranded or single stranded DNA or RNA, or a modification or derivative thereof which, for example, inhibit the expression of the SCD4 gene or the activity of SCD4 and includes, without limitation, antisense nucleic acids, aptamers, siRNAs (small interfering RNAs) and ribozymes.

The inhibitor may be selected from the group consisting of antibodies, antisense oligonucleotides, siRNA, low molecular weight molecules (LMWs), binding peptides, aptamers, ribozymes and peptidomimetics.

LMWs are molecules which are not proteins, peptides, antibodies or nucleic acids, and which exhibit a molecular weight of less than 5000 Da, preferably less than 2000 Da, more preferably less than 2000 Da, most preferably less than 500 Da. Such LMWs may be identified in High-Through-Put procedures starting from libraries. Such methods are known in the art and are discussed in detail below.

These nucleic acids can be directly administered to a cell, or which can be produced intracellularly by transcription of exogenous, introduced sequences.

An "antisense" nucleic acid as used herein refers to a nucleic acid capable of hybridizing to a sequence-specific portion of an protein encoding RNA (preferably mRNA) by virtue of some sequence complementarity. The antisense nucleic acid may be complementary to a coding and/or noncoding region of an mRNA. Such antisense nucleic acids that inhibit protein expression or activity have utility as therapeutics, and can be used in the treatment or prevention of disorders as described herein.

The antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides.

The nucleic acids, e.g. the antisense nucleic acids or siRNAs, can be synthesized chemically, e.g. in accordance with the phosphotriester method (see, for example, Uhlmann, E. & Peyman, A. (1990) Chemical Reviews, 90, 543-584). Aptamers are nucleic acids which bind with high affinity to a polypeptide, here SCD4. Aptamers can be isolated by selection methods such as SELEX (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability.

Nucleic acids may be degraded by endonucleases or exonucleases, in particular by DNases and RNases which can be found in the cell. It is, therefore, advantageous to modify the nucleic acids in order to stabilize them against degradation, thereby ensuring that a high concentration of the nucleic acid is maintained in the cell over a long period of time (Beigelman et al. (1995) Nucleic Acids Res. 23:3989-94; WO 95/11910; WO 98/37240; WO 97/29116). Typically, such a stabilization can be obtained by introducing one or more internucleotide phosphorus groups or by introducing one or more non-phosphorus internucleotides.

Suitable modified internucleotides are compiled in Uhlmann and Peyman (1990), supra (see also Beigelman et al. (1995) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention. In general, the oligonucleotide can be modified at the base moiety, sugar moiety, or phosphate backbone.

The oligonucleotide may include other appending groups such as peptides, agents facilitating transport across the cell membrane (see, e.g., Letsinger et al., 1989, Proc. Natl. Acad. Sci. USA 86:6553-6556; Lemaitre et al., 1987, Proc. Natl. Acad. Sci. USA 84:648-652; International Patent Publication No. WO 88/09810) or blood-brain barrier (see, e.g., International Patent Publication No. WO 89/10134), hybridization-triggered cleavage agents (see, e.g., Krol et al., 1988, BioTechniques 6:958-976), or intercalating agents (see, e.g., Zon, 1988, Pharm. Res. 5:539-549).

In detail, the antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, D-galactosylqueosine, inosine, N6-isopentenyladenine, 1-methylguanine, , 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, D-mannosylqueosine, 5N-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

The use of suitable antisense nucleic acids is further described e.g. in Zheng and Kemeny (1995) Clin. Exp. Immunol., 100, 380-2; Nellen and Lichtenstein (1993) Trends Biochem. Sci., 18, 419-23, Stein (1992) Leukemia, 6, 697-74 or Yacyshyn, B. R. et al. (1998) Gastroenterology, 114, 1142).

In yet another embodiment, the oligonucleotide is a 2-a-anomeric oligonucleotide. An a-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, e.g., a peptide, hybridization-triggered cross-linking agent, transport agent, hybridization-triggered cleavage agent, etc.

Throughout the invention, oligonucleotides of the invention may be synthesized by standard methods known in the art, e.g., by use of an automated DNA synthesizer (such as are commercially avail-able from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. USA 85:7448-7451), etc.

In a specific embodiment, the antisense oligonucleotides comprise catalytic RNAs, or ribozymes (see, e.g., International Patent Publication No. WO 90/11364; Sarver et al., 1990, Science 247:1222-1225). In another embodiment, the oligonucleotide is a 2'-0-methylribonucleotide (Inoue et al., 1987, Nucl. Acids Res. 15:6131-6148), or a chimeric RNA-DNA analog (Inoue et al., 1987, FEBS Lett. 215:327-330).

In an alternative embodiment, the antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the protein. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells. Expression of the sequences encoding the antisense RNAs can be by any promoter known in the art to act in mammalian, preferably human, cells. Such promoters can be inducible or constitutive. Such promoters include, but are not limited to, the SV40 early promoter region (Bernoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. USA 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a protein gene, preferably a human gene, more preferably the human SCD4 gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

The production and use of siRNAs as tools for RNA interference in the process to down regulate or to switch off gene expression, here SCD4 gene expression, is e.g. described in Elbashir, S. M. et al. (2001) Genes Dev., 15, 188 or Elbashir, S. M. et al. (2001) Nature, 411, 494. Preferably, siRNAs exhibit a length of less than 30 nucleotides, wherein the identity stretch of the sense strang of the siRNA is preferably at least 19 nucleotides.

Ribozymes are also suitable tools to inhibit the translation of nucleic acids, here the SCD4 gene, because they are able to specifically bind and cut the mRNAs. They are e.g. described in Amarzguioui et al. (1998) Cell. Mol. Life Sci., 54, 1175-202; Vaish et al. (1998) Nucleic Acids Res., 26, 5237-42; Persidis (1997) Nat. Biotechnol., 15, 921-2 or Couture and Stinchcomb (1996) Trends Genet., 12, 510-5.

Pharmaceutical compositions of the invention, comprising an effective amount of a nucleic acid in a pharmaceutically acceptable carrier, can be administered to a patient having a disease or disorder that is of a type that expresses or overexpresses SCD4.

The amount of the nucleic acid that will be effective in the treatment of a particular disorder or condition will depend on the nature of the disorder or condition, and can be determined by standard clinical techniques. Where possible, it is desirable to determine the nucleic acid cytotoxicity in vitro, and then in useful animal model systems, prior to testing and use in humans.

In a specific embodiment, pharmaceutical compositions comprising nucleic acids are administered via liposomes, microparticles, or microcapsules. In various embodiments of the invention, it may be useful to use such compositions to achieve sustained release of the nucleic acids. In a specific embodiment, it may be desirable to utilize liposomes targeted via antibodies to specific identifiable central nervous system cell types (Leonetti et al., 1990, Proc. Natl. Acad. Sci. U.S.A. 87:2448-2451; Renneisen et al., 1990, J. Biol. Chem. 265:16337-16342).

The term "binding protein" or "binding peptide" refers to a class of proteins or peptides which bind and inhibit SCD4, and includes, without limitation, polyclonal or monoclonal antibodies, antibody fragments and protein scaffolds directed against SCD4.

According to the present invention the term antibody or antibody fragment is also understood as meaning antibodies or antigen-binding parts thereof, which have been prepared recombinantly and, where appropriate, modified, such as chimaeric antibodies, humanized antibodies, multifunctional antibodies, bispecific or oligospecific antibodies, single-stranded antibodies and F(ab) or F(ab)₂ fragments (see, for example, EP-B1-0 368 684, US 4,816,567, US 4,816,397, WO 88/01649, WO 93/06213 or WO 98/24884), preferably produced with the help of a FAB expression library.

As an alternative to the classical antibodies it is also possible, for example, to use protein scaffolds against SCD4, e.g. anticalins which are based on lipocalin (Beste et al. (1999) Proc. Natl. Acad. Sci. USA, 96, 1898-1903). The natural ligand-binding sites of the lipocalins, for example the retinol-binding protein or the bilin-binding protein, can be altered, for example by means of a "combinatorial protein design" approach, in such a way that they bind to selected haptens, here to SCD4 (Skerra, 2000, Biochim. Biophys. Acta, 1482, 337-50). Other known protein scaffolds are known as being alternatives to antibodies for molecular recognition (Skerra (2000) J. Mol. Recognit., 13, 167-187).

The procedure for preparing an antibody or antibody fragment is effected in accordance with methods which are well known to the skilled person, e.g. by immunizing a mammal, for example a rabbit, with SCD4, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromato-graphy. Monoclonal antibodies can, for example, be prepared in accordance with the known method of Winter & Milstein (Winter, G. & Milstein, C. (1991) Nature, 349, 293-299).

In detail, polyclonal antibodies can be prepared as described above by immunizing a suitable subject with a polypeptide as an immunogen. Preferred polyclonal antibody compositions are ones that have been selected for antibodies directed against a polypeptide or polypeptides of the invention. Particularly preferred polyclonal antibody preparations are ones that contain only antibodies directed against a given polypeptide or polypeptides. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes; thereby generating a substantially purified antibody composition, i.e., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein, 1975, Nature 256:495-497, the human B cell hybridoma technique (Kozbor et al., 1983, Immunol. Today 4:72), the EBV-hybridoma technique (Cole et al., 1985, Monoclonal Antibodies and Cancer Therapy, Alan R. Liss, Inc., pp. 77-96) or trioma techniques. The technology for producing hybridomas is well known (see generally Current Protocols in Immunology 1994, Coligan et al. (eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, Bio/Technology 9:1370-1372; Hay et al., 1992, Hum. Antibod. Hybridomas 3:81-85; Huse et al., 1989, Science 246:1275-1281; Griffiths et al., 1993, EMBO J. 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, e.g., Cabilly et al., U.S. Patent No. 4,816,567; and Boss et al., U.S. Patent No. 4,816,397, which are incorporated herein by reference in their entirety.) Humanized antibodies are antibody molecules from non-human species having one or more complementarily determining regions (CDRs) from the non-human species and a framework region from a human immunoglobulin molecule. (See, e.g., -Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559); Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar, 1995, Int. Rev. Immunol. 13:65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope. (Jespers et al., 1994, Bio/technology 12:899-903).

Antibody fragments that contain the idiotypes of a protein, in particular SCD4, can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating, the antibody molecular with papain and a reducing agent; and Fv fragments.

In the production of antibodies, screening for the desired antibody can be accomplished by techniques known in the art, e.g., ELISA (enzyme-linked immunosorbent assay). To select antibodies specific to a particular domain of the protein, or a derivative thereof, one may assay generated hybridomas for a product that binds to the fragment of the protein, or a derivative thereof, that contains such a domain.

The foregoing antibodies can be used in methods known in the art relating to the localization and/or quantification of the given protein or proteins, e.g., for imaging these proteins, measuring levels thereof in appropriate physiological samples (by immunoassay), in diagnostic methods, etc. This hold true also for a derivative, or homologue thereof of SCD4.

In a preferred embodiment, the SCD4 inhibitor is an siRNA with the sequence:
AGUACUCAGAGACGGAUGC

As explained above, it has been surprisingly found in the context of the present invention that SCD4 lowers or attenuates secretion of Abeta-42. Thus, it directly or indirectly regulates beta-secretase and/or gamma secretase activity. Therefore, the inhibitor modulates the activity of beta-secretase and/or gamma secretase.

In the context of the present invention, "modulating the activity of gamma secretase and/or beta secretase" means that the activity is reduced in that less or no product is formed (partial or complete inhibition) or that the respective enzyme produces a different product (in the case of gamma secretase e.g. Abeta-40 instead of Abeta-42) or that the relative quantities of the products are different (in the case of gamma secretase e.g. more Abeta-40 than Abeta-42).

Throughout the invention, the term "modulating the activity of gamma secretase and/or beta secretase" includes that the activity of the enzyme is modulated directly or indirectly. That means that the SCD4 modulator may either bind also directly to the enzyme or, more preferred, may exert an influence on SCD4 which in turn, e.g. by protein-protein interactions or by signal transduction or via small metabolites, modulates the activity of the enzyme.

Furthermore, it is included that the modulator modulates either gamma secretase or beta secretase or the activity of both enzymes.

Throughout the invention, it is preferred that the beta secretase modulator inhibits the activity of beta secretase either completely or partially.

With respect to the modulator of gamma secretase activity, it is preferred that this modulator inhibits gamma secretase activity. However, it is also preferred that the activity of gamma secretase is shifted in a way that more Abeta-40 and/or Abeta-38 is produced instead of Abeta-42.

Gamma secretase activity can e.g. measured by determining APP processing, e.g. by determining whether Abeta-40 or Abeta-42 is produced (see Example-section, infra).

To measure BACE1 activity, changes of the ratio between alpha- and beta-C-terminal APP fragments can be analyzed by Western Blotting (Blasko et al., J Neural Transm 111, 523); additional examples for BACE1 activity assays include but are not limited to: use of a cyclized enzyme donor peptide containing a BACE1 cleavage site to reconstitute and measure beta-galactosidase reporter activity (Naqvi et al., J Biomol Screen. 9, 398); use of quenched fluorimetric peptide substrates and fluorescence measurements (Andrau et al., J.Biol Chem 278, 25859); use of cell-based assays utilizing recombinant chimeric proteins, in which an enzyme (such as alkaline phosphatase) is linked via a stretch of amino acids, that contain the BACE1 recognition sequence, to a Golgi-resident protein (Oh et al., Anal Biochem, 323, 7); fluorescence resonance energy transfer (FRET)-based assays (Kennedy et al., Anal Biochen 319, 49); a cellular growth selection system in yeast (Luthi et al., Biochim Biophys Acta 1620, 167).

Preferably, the neurodegenerative disease is Alzheimer's disease.

It is described that the SCD4 interacting molecule may be used to prepare a pharmaceutical composition.

Therefore, the invention describes pharmaceutical compositions, which may be administered to a subject in an effective amount. In a preferred aspect, the therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a therapeutic e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the therapeutic can be delivered via a controlled release system. In one embodiment, a pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a specific embodiment where the therapeutic is a nucleic acid, preferably encoding a protein therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid therapeutic can be introduced intracellularly and incorporated by homologous recombination within host cell DNA for expression.

In general, the pharmaceutical compositions comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

The composition may be formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

The invention also describes a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits can also contain expression vectors encoding SCD4 or an interacting or binding peptide or polypeptide, which can be used to expressed SCD4 or the respective interacting or binding peptide or polypeptide. Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

The invention further describes a method of treatment, wherein an effective amount of a SCD4 interacting molecule or inhibitor or of a pharmaceutical composition is administered to a subject suffering from a neurodegenerative disease, preferably Alzheimer's disease.

With respect to this method, all embodiments apply given above for the use of the invention.

The invention further relates to a method for identifying a gamma secretase modulator and/or beta-secretase modulator, comprising the following steps:
a. identifying of a SCD4-interacting molecule by determining whether a given test compound is a SCD4-interacting molecule,
b. determining whether the SCD4-interacting molecule of step a) is capable of modulating gamma secretase activity or beta-secretase activity.

In a preferred embodiment of the invention, in step a) the test compound is brought into contact with SCD4 and the interaction of SCD4 with the test compound is determined. Preferably, it is measured whether the candidate molecule is bound to SCD4.

The method of the invention is preferably performed in the context of a high throughput assay. Such assays are known to the person skilled in the art.

Test or candidate molecules to be screened can be provided as mixtures of a limited number of specified compounds, or as compound libraries, peptide libraries and the like. Agents/molecules to be screened may also include all forms of antisera, antisense nucleic acids, etc., that can modulate SCD4 activity or expression. Exemplary candidate molecules and libraries for screening are set forth below.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852;.U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a SCD4 immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, SCD4 fragments and/or analogs, especially peptidomimetics, are screened for activity as competitive or non-competitive inhibitors of presence of SCD4 (e.g. SCD4 expression or stability) or, particularly, SCD4 activity in the cell.

In one embodiment, agents that modulate (i.e., inhibit or activate) SCD4 activity can be screened for using a Abeta-42 secretion assay, wherein agents are screened for their ability to modulate SCD4 activity under aqueous, or physiological, conditions in which SCD4 is active in absence of the agent to be tested. Preferably, the candidate agents are agents that interact with or bind to SCD4. Agents that interfere with the secretion of Abeta-42 are identified as inhibitors of SCD4 activity. Agents that promote the secretion of Abeta-42 are identified as activators of SCD4.

Preferably, a two-step procedure can be used involving (a) identifying modulators in an SCD4 activity assay (e.g. an assay measuring the Delta-9 desaturase activity of SCD4, e.g. as described in Obukowicz MG et al., supra), and (b) testing the modulators for Abeta-42 lowering or attenuating activity.

Methods for screening, particularly methods for screening for agents that bind to SCD4, may involve labeling SCD4 with radioligands (e.g., ¹²⁵I or ³H), magnetic ligands (e.g., paramagnetic beads covalently attached to photobiotin acetate), fluorescent ligands (e.g., fluorescein or rhodamine), or enzyme ligands (e.g., luciferase or β-galactosidase). The reactants that bind in solution can then be isolated by one of many techniques known in the art, including but not restricted to, co-immunoprecipitation of the labeled protein using antisera against the unlabeled binding partner (or labeled binding partner with a distinguishable marker from that used on the second labeled protein), immunoaffinity chromatography, size exclusion chromatography, and gradient density centrifugation. In one embodiment, the labeled binding partner is a small fragment or peptidomimetic that is not retained by a commercially available filter. Upon binding, the labeled species is then unable to pass through the filter, providing for a simple assay of binding.

Methods commonly known in the art are used to label at least one of the proteins or polypeptides. Suitable labeling methods include, but are not limited to, radiolabeling by incorporation of radiolabeled amino acids, e.g., ³H-leucine or ³⁵S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where one of the binding partners is immobilized, e.g., as described infra, the free species is labeled. Where neither of the interacting species is immobilized, each can be labeled with a distinguishable marker such that isolation of both partners can be followed to provide for more accurate quantification, and to distinguish the formation e.g. of homomeric from heteromeric binding. Methods that utilize accessory proteins that bind to one of the modified partners to improve the sensitivity of detection, increase the stability of the binding, etc., are provided.

The same labeling methods as described above may also be used to label e.g. APP, Abeta-40, or Abeta-42, for example to determine the amount of secreted Abeta-40 or Abeta-42 in an Abeta secretion assay.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular binding can be assayed using routine protein binding assays to determine optimal binding conditions for reproducible binding.

The physical parameters of binding can be analyzed by quantification of binding using assay methods specific for the label used, e.g., liquid scintillation counting for radioactivity detection, enzyme activity for enzyme-labeled moieties, etc. The reaction results are then analyzed utilizing Scatchard analysis, Hill analysis, and other methods commonly known in the arts (see, e.g., Proteins, Structures, and Molecular Principles, 2^{nd} Edition (1993) Creighton, Ed., W.H. Freeman and Company, New York).

In a second common approach to binding assays, one of the binding species is immobilized on a filter, in a microtiter plate well, in a test, tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

Assays of agents (including cell extracts or a library pool) which compete for binding of a given molecule to SCD4 are provided to screen for competitors, enhancers, or agents with specifically desired binding characteristics (e.g. lower or higher affinity) compared to a given binding partner. Again, either the molecule or SCD4 can be labeled by any means (e.g., those means described above).

In specific embodiments, blocking agents to inhibit non-specific binding of reagents to other proteins, or absorptive losses of reagents to plastics, immobilization matrices, etc., are included in the assay mixture. Blocking agents include, but are not restricted to bovine serum albumin, casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow specific binding.

After binding is performed, unbound, labeled agent is removed in the supernatant, and the immobilized protein (or, if applicable, the immobilized agent) retaining any bound, labeled agent is washed extensively. The amount of bound label is then quantified using standard methods in the art to detect the label as described, supra.

In another specific embodiments screening for modulators of the protein as provided herein can be carried out by attaching those and/or the antibodies as provided herein to a solid carrier.

The preparation of such an array containing different types of proteins (including antibodies) is well known in the art and is apparent to a person skilled in the art (see e.g. Ekins et al., 1989, J. Pharm. Biomed. Anal. 7:155-168; Mitchell. et al. 2002, Nature Biotechnol. 20:225-229; Petricoin et al., 2002, Lancet 359:572-577; Templin et al., 2001, Trends Biotechnol. 20:160-166; Wilson and Nock, 2001, Curr. Opin. Chem. Biol. 6:81-85; Lee et al., 2002 Science 295:1702-1705; MacBeath and Schreiber, 2000, Science 289:1760; Blawas and, Reichert, 1998, Biomaterials 19:595; Kane et al., 1999, Biomaterials 20:2363; Chen et al., 1997, Science 276:1425; Vaugham et al., 1996, Nature Biotechnol. 14:309-314; Mahler et al., 1997, Immunotechnology 3:31-43; Roberts et al., 1999, Curr. Opin. Chem. Biol. 3:268-273; Nord et al., 1997, Nature Biotechnol. 15:772-777; Nord et al., 2001, Eur. J. Biochem. 268:4269-4277; Brody and Gold, 2000, Rev. Mol. Biotechnol. 74:5-13; Karlstroem and Nygren, 2001, Anal. Biochem. 295:22-30; Nelson et al., 2000, Electrophoresis 21:1155-1163; Honore et al., 2001, Expert Rev. Mol. Diagn. 3:265-274; Albala, 2001, Expert Rev. Mol. Diagn. 2:145-152, Figeys and Pinto, 2001, Electrophoresis 2:208-216 and references in the publications listed here).

Proteins or other agents can be attached to an array by different means as will be apparent to a person skilled in the art. Proteins can for example be added to the array via a TAP-tag (as described in WO/0009716 and in Rigaut et al., 1999, Nature Biotechnol. 10:1030-1032) after the purification step or by another suitable purification scheme as will be apparent to a person skilled in the art.

Optionally, functional assays as will be apparent to a person skilled in the art, some of which are exemplarily provided herein, can be performed to check the integrity of the protein bound to the matrix.

Optionally, the attachment of the proteins or antibody as outlined above can be further monitored by various methods apparent to a person skilled in the art. Those include, but are not limited to surface plasmon resonance (see e.g. McDonnel, 2001, Curr. Opin. Chem. Biol. 5:572-577; Lee, 2001, Trends Biotechnol. 19:217-222; Weinberger et al., 2000, 1:395-416; Pearson et al., 2000, Ann. Clin. Biochem. 37:119-145; Vely et al., 2000, Methods Mol. Biol. 121:313-321; Slepak, 2000, J. Mol Recognit. 13:20-26.

Exemplary assays useful for measuring the production of Abeta-40 and Abeta-42 peptides by ELISA include but are not limited to those described in Vassar R et al., 1999, Science, 286:735-41.

Exemplary assays useful for measuring the production of C-terminal APP fragments in cell lines or transgenic animals by Western Blot include but are not limited to those described in Yan R et al., 1999, Nature, 402:533-7.

Exemplary assays useful for measuring the proteolytic activity of beta- or gamma secretases towards bacterially expressed APP fragments in vitro (e.g. by modifying the expression of SCD4 proteins in cells by means of RNAi (siRNA) and/or plasmids encoding the SCD4 protein include but are not limited to those described in Tian G et al., 2002, J Biol Chem, 277:31499-505.

Exemplary assays useful for measuring transactivation of a Gal4-driven reporter gene (e.g. by modifying the expression of SCD4 by means of RNAi (siRNA) and/or plasmids encoding SCD4 protein, include but are not limited to those described in Cao X et al., 2001, Science, 293:115-20.

Any molecule known in the art can be tested for its ability to be an interacting molecule or inhibitor according to the present invention. Candidate molecules can be directly provided to a cell expressing the SCD4, or, in the case of candidate proteins, can be provided by providing their encoding nucleic acids under conditions in which the nucleic acids are recombinantly expressed to produce the candidate protein.

The method of the invention is well suited to screen chemical libraries for molecules which modulate, e.g., inhibit, antagonize, or agonize, the amount or activity the protein, in particular of SCD4. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention. Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et.al., 1994, J. Med. Chem. 37:1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of, -carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the α carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, amino isobutyric acid, 4-aminobutyric acid, Abu, 2-amino butyric acid; -Abu, -Ahx, 6-amino hexanoic acid; Aib, 2-amino isobutyric acid; 3-amino propionic acid; ornithine; norleucine; norvaline, hydroxyproline, sarcosine, citrulline, cysteic acid, t-butylglycine, t-butylalanine, phenylglycine, cyclohexylalanine, β-alanine, designer amino acids such as β-methyl amino acids, C-methyl amino acids, N-methyl amino acids, fluoro-amino acids and amino acid analogs in general. Furthermore, the amino acid can be D (dextrorotary) or L (levorotary).

In a specific embodiment, fragments and/or analogs of proteins of the invention, especially peptidomimetics, are screened for activity as competitive or non-competitive inhibitors of SCD4 expression (e.g. stability) or activity.

In another embodiment of the present invention, combinatorial chemistry can be used to identify modulators of SCD4. Combinatorial chemistry is capable of creating libraries containing hundreds of thousands of compounds, many of which may be structurally similar. While high throughput screening programs are capable of screening these vast libraries for affinity for known targets, new approaches have been developed that achieve libraries of smaller dimension but which provide maximum chemical diversity. (See e.g., Matter, 1997, J. Med. Chem. 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest, in particular of SCD4. The fingerprints are compared with fingerprints obtained from other compounds known to react with the protein of interest to predict whether the library compound might similarly react. For example, rather than testing every ligand in a large library for interaction with a protein, only those ligands having a fingerprint similar to other compounds known to have that activity could be tested. (See, e.g., Kauvar et al., 1995, Chem. Biol. 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, Kurtz, Stanker and Skerritt (eds), 1995, AOAC: Washington, D.C., 305-312).

Kay et al. (1993, Gene 128:59-65) disclosed a method of constructing peptide libraries that encode peptides of totally random sequence that are longer than those of any prior conventional libraries. The libraries disclosed in Kay et al. encode totally synthetic random peptides of greater than about 20 amino acids in length. Such libraries can be advantageously screened to identify protein modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

In a preferred embodiment, the interaction of the test compound with SCD4 results in an inhibition of SCD4 activity.

According to a preferred embodiment, in step b) the ability of the gamma secretase to cleave APP is measured. This can be measured as indicated above.

According to another preferred embodiment, in step b) the ability of the SCD4-interacting molecule to lower or attenuate the secretion of Abeta-42 is measured.

Further, the invention also describes a method for preparing a pharmaceutical composition for the treatment of neurodegenerative diseases, preferably Alzheimer's disease, comprising the following steps:
a) identifying a gamma secretase modulator and/or beta-secretase modulator, preferably inhibitor, according to the method of the invention, and
b) formulating the gamma secretase and/or beta-secretase modulator, preferably inhibitor, to a pharmaceutical composition.

With respect to the pharmaceutical composition, all embodiments as indicated above apply also here. This method may further comprise the step of mixing the identified molecule with a pharmaceutically acceptable carrier as explained above.

The invention also describes a pharmaceutical composition comprising a SCD4 inhibitor as defined above.

Furthermore, the invention also describes a pharmaceutical composition obtainable by the above method for the preparation of a pharmaceutical composition.

The invention is also describes the pharmaceutical composition for the treatment of a neurodegenerative disease such as Alzheimer's disease and related neurodegenerative disorders.

The invention also describes a method for treating or preventing a neurodegenerative disease, preferably Alzheimer's disease, comprising administering to a subject in need of such treatment or prevention a therapeutically effective amount of a pharmaceutical composition of as described above

With respect to that method, all embodiments as described above for the use of the invention also apply.

The invention also relates to the use of a SCD4 inhibitor for the modulation, preferably inhibition of beta secretase and/or gamma secretase activity in vitro wherein the inhibitor is selected from the group consisting of antisense oligonucleotides, si RNA and ribozymes. For example, it is encompassed within the present invention to modulate, preferably inhibit beta secretase and/or gamma secretase activity in cell cultures by the SCD4 inhibitor. All embodiments with respect to the SCD4 inhibitor as described above also apply to this use of the invention.

The invention is further illustrated but not limited in any way by the following figures and examples:
Fig. 1:
   SCD4 is very highly expressed in human brain.
   5 µg of total RNA from various human tissue sources (Clontech) was reverse transcribed. Equal amounts of cDNAs from each tissue and SCD4-specific primers were utilized for determination of relative expression levels of SCD4 by quantitative PCR. Three independent experiments were performed and all values were normalized to a human reference RNA (Stratagene).
Fig. 2:
   siRNA-mediated knock-down of SCD4 expression attenuates secretion of Aβ1-42.
   (left panel) siRNAs directed against BACE1, SCD4 or Luc3 were transfected into H4 neuroglioma cells over-expressing mutant APPsw. 48h after transfection growth medium was removed and cells were incubated over night in serum-free medium. Supernatants were collected and levels of Aβ1-42 determined by ELISA (Innogenetics). At least three independent experiments were performed in duplicate.
   (right panel) siRNA directed against SCD4 specifically reduces mRNA levels. Total RNA was prepared from H4/APPsw cells transfected with siRNA directed against either Luc3 or SCD4. After reverse transcription, relative amounts of SCD4 transcripts were determined by quantitative PCR. At least two independent experiments were performed.
Fig. 3:
   Amino acid sequence of human SCD4 (SCD4/Hypothetical Protein FL J21032), depicted in the one-letter-code.

### EXAMPLES:

The following examples refer to all embodiments of the invention and especially to the embodiments as claimed in the claims.

### EXAMPLE 1: determination of SCD-4 tissue expression levels

To assess whether SCD-4 qualified as a potential target for AD, we investigated whether it was expressed in human brain. To that end, we determined its expression levels in various tissues by reverse-transcription polymerase chain reaction (RT-PCR). Briefly, 5 µg of total RNA from various human tissue sources (Clontech) was reverse transcribed using standard procedures. Equal amounts of cDNAs from each tissue and SCD4-specific primers were utilized for determination of relative expression levels of SCD-4 by quantitative PCR following manufacturer's instructions. All values were normalized to a human reference RNA (Stratagene).

### EXAMPLE 2: siRNA-Inhibition of SCD4

A RNAi gene expression perturbation strategy was employed for functional validation of SCD-4 as an effector of APP processing: An siRNAs directed against SCD-4 as well as siRNAs directed against BACE1 or Luc3 into was transfected into SKNBE2 neuroblastoma or H4 neuroglioma cells. siRNAs for human SCD-4 were synthesized by Dharmacon Research Inc.
The sequence of the siRNA used for SCD-4 is: AGUACUCAGAGACGGAUGC.

Transfection of SK-N-BE2 cells was performed using LipofectAMINE 2000 (Invitrogen) following the manufacturer's instructions. Briefly, the cells were seeded at a density of 1.0 x 10⁴ cells in a final volume of 85 µl per 96-well 12-16 hrs prior to transfection. 25 nM of siRNAs were mixed with 8 µl Opti-MEM buffer (Gibco) and 60 ng carrier DNA, and the mixture was incubated for 20 minutes at room temperature before addition to the cells. 16 and 48 hrs post-transfection medium was replaced with 100 µl or 200 µl growth medium with or without serum, respectively. 72 hrs post-transfection, 100 µl supernatants were harvested for Aβ42 ELISA. The assay was performed following the manufacturer's instructions (Innogenetics).

Transfection of H4 cells was performed using RNAiFect. (Qiagen) following the manufacturer's instructions. Briefly, the cells were seeded at a density of 1.0 x 10⁴ cells in a final volume of 100 µl per 96-well 12-16 hrs prior to transfection. 270 nM (0,375 µg) of siRNAs were mixed with 25 µl EC-R buffer and 2,3 µl of RNAiFect and incubated for 15 minutes at room temperature before addition to the cells. Medium on cells was replaced with 75 µl of fresh growth medium. 5 hrs post-transfection the cells were washed once with growth medium and 100 µl were added for further cultivation. 48 hrs post-transfection medium was replaced with 200 µl serum-free growth medium 72 hrs post-transfection 100 µl supernatants were harvested for Aβ42 ELISA. The assay was performed following the manufacturer's instructions (Innogenetics).

Knockdown efficiency of selected siRNAs was assessed at the protein level by co-transfecting siRNAs and corresponding TAP-tagged cDNA expression vectors or by using cell lines stably expressing the respective tagged protein of interest. 48 hrs post-transfection extracts were prepared, proteins separated by SDS-PAGE and transferred to nitrocellulose. Western blots were probed with antibodies directed against the tag and tubulin.

We noticed that like siRNAs directed against the known effector of APP processing, BACE1, the siRNA targeting SCD-4 caused significant attenuation of Aβ1-42 secretion, whereas the Luc3 siRNA had no effect.

Thus, we could show that SCD-4 plays a functional role in the processing of APP. It was shown that by inhibiting SCD-4, the production of the Aβ1-42 peptide could be reduced.

We confirmed that the SCD-4 siRNAs did indeed interfere with expression of the desaturase on the mRNA level by RT-PCR analysis as described above.

### EXAMPLE 3: Determination of SCD-4-activity

### a) Rat liver microsomal assay

Adapted for measurement of SCD-4 activity from: (Obukowicz MG, Raz A, Pyla PD, Rico JG, Wendling JM, Needleman P (1998a) Identification and characterization of a novel delta6/delta5 fatty acid desaturase inhibitor as a potential anti-inflammatory agent. Biochem. Pharmacol. 1;55(7): 1045-58; Obukowicz MG, Welsch DJ, Salsgiver WJ, Martin-Berger CL, Chinn KS, Duffin KL, Raz A, Needleman P (1998b) Novel, selective delta6 or delta5 fatty acid desaturase inhibitors as antiinflammatory agents in mice. J. Pharmacol. Exp. Ther. 287(1):157-66)

Rat microsomal membranes are obtained by standard biochemical fractionation procedures.
In a 48-well plate the following components are mixed: a) 150µl buffer/cofactors (250 mM sucrose, 150 mM KC1, 40 mM NaF, 1.3 mM ATP, 1 mg/ml MgCl2 * 5 H₂0, 1.5 mM reduced glutathione, 60 µM reduced CoA, 330 µM nicotinamide, 670 µg/ml NADH, 100 mM sodium phosphate,pH 7.4); b) 50µl rat liver micro-somes (~500 µg total protein); c) 2.2 µl test compound (DMSO stock; 1% final DMSO concentration); d) 20µl (0.05 µCi) ¹⁴C-Fatty Acid Substrates.
The assay allows for simultaneous measurement of SCD1 and SCD4 activity (Δ9 desaturase). The substrate for these enzymatic activities is stearic acid (¹⁴C18:0).
Samples are incubated at 37°C for 1 hr, and then reactions are stopped and fatty acid ester linkages hydrolyzed by incubation with 200µl 2.5N KOH in methanol:water (4:1) for 4 h at 65°C. Free fatty acids are protonated with 280 µl formic acid and extracted into organic phase (700 µl hexane). 200µl from hexane layer are analyzed on AgNO₃-thin-layer chromatography (TLC) plates. Plates are dried over night and activity is quantified by phosphoimager. As an alternative to TLC analysis, separation of samples could be achieved by HPLC.

### b) Cellular assay

A cell line expressing high levels of SCD4 is utilized. Cells are adapted to grow in a suitable serum-free medium containing SCD4 substrates (such as 10 µM stearic acid/15 µM fatty acid-free BSA). To measure SCD4 activity 2x10⁵ cells of are plated per 48-well and then incubated in medium containing 10 µM of a suitable substrate (such as stearic acid (¹⁴C18:0)). To terminate fatty acid metabolism, the cell layer is washed with PBS and 200 µl 2.5N KOH in methanol:water (4:1) are added. Samples are further processed as described above.

### c) High-troughput screening assays using fatty acid synthetic enzymes (s. WO-03/019146, p.27 ff.)

The assay utilizes position-specifically tritiated fatty acyl-CoA esters in a microsomal assay format (see above). The method detects the release of tritiated water and circumvents the requirement of TLC- or HPLC analysis of ¹⁴C-labeled fatty acids.

Briefly, the following components are mixed (total volume: 100 µl): 2 µl unlabeled 1.5 mM fatty acyl CoA; 1 µl tritiated fatty acyl CoA, 10 µl 20 mM NADH, compounds from DMSO stock, 67 µl 100 mM phosphate buffer, pH 7.2. 80 µl of this mix are added to 20 µl of microsomes (∼20 µg total protein) and reaction is allowed to proceed for 5-30 min at RT. 10µl 6% perchloric acid are added to stop the reaction. To sediment unused tritiated substrate, samples are vortexed with 100 µl charcoal suspension and centrifuged at 13,000rpm for 10min at 4°C. 400µl of supernatant is analyzed in a liquid scintillation counter.

## Claims

1. Use of a SCD4 inhibitor for the preparation of a pharmaceutical composition for the treatment of Alzheimer's disease, wherein the inhibitor is selected from the group consisting of antisense oligonucleotides, siRNA and ribozymes, and wherein the inhibitor modulates the activity of gamma secretase and/or beta secretase.

2. A method for identifying a gamma secretase and/or a beta secretase modulator, comprising the following steps:
a. identifying of a SCD4-interacting molecule by determining whether a given test compound is a SCD4-interacting molecule,
b. determining whether the SCD4-interacting molecule of step a) is capable of modulating gamma secretase and/or beta secretase activity.

3. The method of claim 2, wherein in step a) the test compound is brought into contact with SCD4 and the interaction of SCD4 with the test compound is determined.

4. The method of claim 3, wherein the interaction of the test compound with SCD4 results in an inhibition of SCD4 activity.

5. The method of any of claims 2 to 4, wherein in step b) the ability of the gamma secretase and/or the beta secrease to cleave APP is measured.

6. Use of a SCD4 inhibitor for the modulation of beta secretase and/or gamma secretase activity in vitro, wherein the inhibitor is selected from the group consisting of antisense oligonucleotides, siRNA, and ribozymes.

## Patentansprüche

1. Verwendung eines SCD4-Inhibitors fiir die Herstellung einer pharmazeutischen Zusammensetzung für die Behandlung der Alzheimerkrankheit, wobei der Inhibitor ausgewählt ist aus der Gruppe bestehend aus Antisense-Oligonukleotiden, siRNA und Ribozymen, und wobei der Inhibior die Aktivität von gamma-Sekretase und/oder beta-Sekretase moduliert.

2. Verfahren zum Identifizieren eines gamma-Sekretase und/oder eines beta-Sekretase Modulators, umfassend die folgenden Schritte:
a. Identifizieren eines SCD4-interagierenden Moleküls durch Bestimmen, ob eine gegebene Test-Verbindung ein SCD4-interagierendes Molekül ist.
b. Bestimmen, ob das SCD4-interagierende Molekül von Schritt a) imstande ist, gamma-Sekretase und/oder beta-Sekretase zu modulieren.

3. Verfahren nach Anspruch 2, wobei in Schritt a) die Test-Verbindung mit SCD4 in Kontakt gebracht wird, und die Interaktion von SCD4 mit der Test-Verbindung bestimmt wird.

4. Verfahren nach Anspruch 3, wobei die Interaktion der Test-Verbindung mit SCD4 in einer Inhibition der SCD4-Aktivität resultiert.

5. Verfahren nach irgendeinem der Ansprüche 2 bis 4, wobei in Schritt b) die Fähigkeit der gamma-Sekretase und/oder der beta-Sekretase, APP zu spalten, gemessen wird.

6. Verwendung eines SCD4-Inhibitors für die Modulation der beta-Sekretase- und/oder gamma-Sekretase Aktivität in vitro, wobei der Inhibitor ausgewählt ist aus der Gruppe bestehend aus Antisense-Oligonukleotiden, siRNA und Ribozymen.

## Revendications

1. Utilisation d'un inhibiteur de SCD4 pour la préparation d'une composition pharmaceutique pour le traitement de la maladie d'Alzheimer, dans laquelle l'inhibiteur est choisi dans le groupe constitué par les oligonucléotides antisens, le siARN et les ribosomes, et dans laquelle l'inhibiteur module l'activité de la gamma-sécrétase et/ou de la bêta-sécrétase.

2. Le procédé d'identification d'un modulateur de la gamma-sécrétase et/ou de la bêta-sécrétase, comprenant les étapes consistant à :
a. identifier une molécule interagissant sur le SCD4 par détermination du fait qu'un composé étudié donné est ou non une molécule interagissant sur le SCD4,
b. déterminer si une molécule interagissant sur le SCD4 de l'étape a) est capable de moduler l'activité de la gamma-sécrétase et/ou de la bêta-sécrétase.

3. Procédé selon la revendication 2, dans lequel dans l'étape a), le composé étudié est mis en contact avec le SCD4 et l'interaction du SCD4 avec le composé étudié est déterminée.

4. Procédé selon la revendication 3, dans lequel l'interaction du composé étudié avec le SCD4 conduit à une inhibition de l'activité du SCD4.

5. Procédé selon l'une quelconque des revendications 2 à 4, dans lequel dans l'étape b), est déterminée la capacité de la gamma-sécrétase et/ou de la bêta-sécrétase à couper l'APP.

6. Utilisation d'un inhibiteur de SCD4 pour la modulation de l'activité de la gamma-sécrétase et/ou de la bêta-sécrétase in vitro, dans laquelle l'inhibiteur est choisi dans le groupe constitué par les oligonucléotides antisens, le siARN et les ribosomes.
